# EUROPÄISCHE PATENTSCHRIFT

(11) **EP 2 563 410 B1**
(45) Veröffentlichungstag und Bekanntmachung des Hinweises auf die Patenterteilung: **18.06.2014**
(21) Anmeldenummer: 11716266.9
(22) Anmeldetag: 26.04.2011
(51) Int. Cl.: A61L 2/025, A61L 2/07, A61L 2/10

(54) **VERFAHREN ZUR MASCHINELLEN REINIGUNG UND DESINFEKTION VON GEGENSTÄNDEN**
METHOD FOR MACHINE-CLEANING AND MACHINE-DISINFECTING OBJECTS
PROCÉDÉ POUR LE NETTOYAGE ET LA DÉSINFECTION À LA MACHINE D'OBJETS

(30) Priorität: 28.04.2010 DE 102010028339
(43) Veröffentlichungstag der Anmeldung: 06.03.2013
(73) Patentinhaber: Saiger, Lothar, 88525 Dürmentingen (DE)
(72) Erfinder: Saiger, Lothar, 88525 Dürmentingen (DE)
(74) Vertreter: Kohler Schmid Möbus
(86) Internationale Anmeldenummer: PCT/EP2011/056595
(87) Internationale Veröffentlichungsnummer: WO 2011/134970

(56) Entgegenhaltungen:
- WO-A1-96/20016
- DE-A1- 2 932 565
- DE-A1- 19 860 290
- US-A1- 2005 220 665

## Beschreibung

Die Erfindung betrifft ein Verfahren zur maschinellen Reinigung und Desinfektion von Gegenständen, insbesondere medizinischem und/oder zahnmedizinischem Instrumentarium und/oder Arbeitsmitteln.

Kontaminierte Medizinprodukte und Arbeitsmittel müssen vor deren Wiederverwendung am Menschen oder Tier gereinigt, desinfiziert und ggf. auch sterilisiert werden. Die rechtlichen Rahmenbedingungen stehen in zahlreichen Hygienegesetzen.

Heute werden dazu "Spülmaschinen" eingesetzt: Diese haben gravierende Nachteile: Unzureichende Reinigungsleistung, Spülschatten, hohe Kosten, Einsatz von gesundheitsschädlichen Chemikalien, hoher Zeitaufwand, Resistenzen. Außerdem sind z.B. aus der Schrift DE 198 60 290 A1 Verfahren bekannt, bei denen zu reinigende Gegenstände in einem Dampfsterilisator in einer Reinigungsflüssigkeit ultraschallbehandelt und nach Ablassen der Reinigungsflüssigkeit mit von außen zugeführtem Dampf sterilisiert werden.

Aufgabe der Erfindung ist es, ein Verfahren zur maschinellen Reinigung und Desinfektion von Gegenständen anzugeben, das eine einfachere, schnellere, zuverlässigere und zudem kostengünstigere Reinigung und Desinfektion von Gegenständen ermöglicht.

Diese Aufgabe wird erfindungsgemäß durch ein Verfahren mit den Merkmalen des unabhängigen Anspruchs 1 gelöst.
Weitere Vorteile und vorteilhafte Ausgestaltungen des Gegenstands der Erfindung sind der Beschreibung, der Zeichnung und den Ansprüchen entnehmbar.
Ein wesentlicher Vorteil des erfindungsgemäßen Verfahren ist, dass damit reproduziere Reinigungs- und Desinfektionsergebnisse entstehen(Validierung).

Auf den Gegenständen anhaftende Verunreinigungen und Keime werden durch den Ultraschall zunächst effizient von den Gegenständen angelöst und in dem Wasser suspendiert, zudem Keimverbände und Verklumpungen im Wasser aufgelöst. Dieses Vorgehen ersetzt eine manuelle Reinigung vollumfänglich. Die Ultraschallreinigung erfolgt auch im Inneren von Gegenständen, sofern die Gegenstände Hohlkörper aufweisen.

Reinigungszusätze, und desinfizierend wirkende Substanzen können zur Wirksamkeitssteigerung zugesetzt werden.

Erfindungsgemäß wird ein definiertes (zur Desinfektion ausreichendes) Restvolumen des bei der Ultraschallreinigung eingesetzten Wassers nach der Ultraschallreinigung im Arbeitsraum verdampft.

Wesentliche erfindungsgemäße Vorteile, im Vergleich zum Erhitzen des gesamten Ultraschallbades sind, dass das geringere Restvolumen schneller erhitzt werden kann und dass durch das Verdampfen höhere Temperaturen als 100 Grad Celsius erreicht werden. Ein externer Dampferzeuger ist nicht erforderlich.
Optimierungen des Desinfektionserfolges können durch Erwärmen des Ultraschallbades, der Dauer der Dampfeinwirkung und der Temperatur des Wasserdampfes erreicht werden.
Nach der Reinigung und Desinfektion können die Gegenstände passiv, durch Verdampfen des Restwassers auf der Oberfläche der Gegenstände und aktiv, beispielsweise durch einen Lüfter getrocknet werden.

Erfindungsgemäß können die Gegenstände während der Ultraschallreinigung mit keimabtötenden UV-Strahlen bestrahlt werden. Dadurch ist gewährleistet, dass im Wasser sowie auf den Gegenständen befindliche Keime bereits im Vorfeld der Dampfdesinfektion der Gegenstände von der UV-Strahlung dezimiert, d.h. abgetötet werden. Das für UV-Strahlen durchlässige Wasser wird dabei durch die UV-Strahlung laufend desinfiziert. Die UV-Applikation bietet zudem den Vorteil fehlender bakterieller Resistenzen. Der mit der kombinierten Ultraschallreinigung und UV-Strahlung erreichte Reinigungs- bzw. Desinfektionsgrad ermöglicht zusammen mit der nachfolgenden Dampfdesinfektion einen synergistischen Desinfektionseffekt.

Nach einer bevorzugten Weiterbildung der Erfindung wird das in dem Arbeitsraum verbleibende Restvolumen des Wassers mittels eines elektrischen Heizelements, insbesondere mittels einer Heizwendel und/oder mittels eines Infrarotstrahlers, in Wasserdampf umgewandelt.
Im Falle der Heizwendel kann das Wasser sehr zügig und ohne technischen Aufwand in Wasserdampf umgewandelt werden.

Bei dem alternativen oder zusätzlichen Einsatz eines oder mehrerer Infrarotstrahler können die Gegenstände, sofern Sie von dem zu verdampfenden Restvolumen des Wassers nicht mehr benetzt sind, vorteilhafter Weise unabhängig von einem Aufheizen des Wassers auf Temperaturen von über 100 ° Celsius, gebracht werden. Dadurch kann der thermische Desinfektionsprozess nochmals zügiger einsetzen.
Ein Lüfter kann die thermische Desinfektion durch Umwälzen der Dampfatmosphäre zusätzlich unterstützen.

Um einen reproduzierbaren Desinfektionsgrad zu gewährleisten, wird die thermische Desinfektion der Gegenstände über einen längeren Zeitraum durchgeführt

Zur thermischen Desinfektion der Gegenstände wird vorzugsweise Wasserdampf mit einer Temperatur von 100° bis 140° Celsius eingesetzt. Temperaturen von ca. 134° Celsius und mehr können insbesondere bei thermoresistenten Keimen eingesetzt werden.

Nach einer Weiterbildung der Erfindung wird bei der thermischen Desinfektion vorzugsweise Stattdampf verwendet.

Die Desinfektion und Reinigung der Gegenstände wird dadurch weiter verbessert und beschleunigt, dass der in dem Arbeitsraum erzeugte Wasserdampf den Gegenständen (zusätzlich) mittels zumindest einer Düse als Dampfstrahl zugeführt wird. Dadurch können die Gegenstände schneller und gleichmäßiger auf eine vorbestimmte Temperatur erhitzt und gleichzeitig zusätzlich gereinigt werden. Die beschriebene Reinigungswirkung entspricht dem Wirkprinzip eines Dampfstrahlgerätes. Diese Vorgehensweise hat den Vorteil, dass durch die gezielte Lenkung des Wasserdampfes der Ausbildung von Luftpolstern, die einen Desinfektionserfolg gefährden könnten, entgegengewirkt wird.

Durch ein Überdruckventil oder durch eine, vorzugsweise in ihrem Strömungsquerschnitt einstellbare Auslassöffnung wird erreicht, dass sich der Druck im Arbeitsraum nicht wesentlich über den Umgebungsluftdruck unkontrolliert erhöht.

Die Gegenstände werden nach einer Weiterbildung der Erfindung vor der Ultraschallreinigung, beim Verdampfen des Restwassers im Arbeitsraum und ggf. auch bei der thermischen Desinfektion mit UV - Strahlen bestrahlt.

Zur effektiven Reinigung von Gegenständen mit einem von außen zugänglichen Hohlraum, insbesondere (zahn-) medizinischen Hand- und Winkelstücken, werden diese nach der Erfindung an eine Spüleinrichtung des Reinigungs- und Desinfektionssystems angeschlossen, wobei die Gegenstände während der Ultraschallreinigung mit dem (flüssigen) Wasser und während der thermischen Desinfektion mit dem Wasserdampf durchgespült werden. Dadurch können die durch Ultraschall von inneren Oberflächen der Gegenstände abgelösten Verunreinigungen und Keime noch effizienter aus den Gegenständen heraus gespült werden. Zugleich wird sichergestellt, dass der Dampf unverzüglich in direkten Kontakt mit den inneren Oberflächen der Gegenstände kommt, so dass auch diese schnell und zuverlässig desinfiziert werden.

Nach der Reinigung werden die Gegenstände, z.B. die medizinischen Instrumente, erhitzt und das in den Hohlräumen befindliche Wasser verdampft vor Ort und desinfiziert sicher und effizient auch schwer zugängliche Hohlräume.

Unabhängig von der Erhitzung des Restwassers im Arbeitsraum kann im Arbeitsraum, getrennt von allen Gerätekomponenten, ein Dampfgenerator vorgesehen sein, der zusätzlich Dampf erzeugen kann und dieser Dampf kann direkt auf die zu desinfizierenden Gegenstände abgeben oder in Hohlräume der zu desinfizierenden Gegenstände eingeblasen werden.

Erfindungsgemäß werden die Reinigungs- und Desinfektionsparameter des Reinigungs- und Desinfektionssystems von einer Steuereinheit gesteuert. Die Reproduzierbarkeit der Reinigungs- und Desinfektionsergebnisse wird durch definierte Programmabläufe gesichert.

Erfindungsgemäß können alle von der Steuereinheit erfassten (Betriebs-) Parameter elektronisch dokumentiert werden und können so jederzeit zur Validierung des Reinigungs- und Desinfektionsprozesses herangezogen werden.

Nachfolgend wird die Erfindung anhand eines in der Zeichnung wiedergegebenen Ausführungsbeispiels näher erläutert.

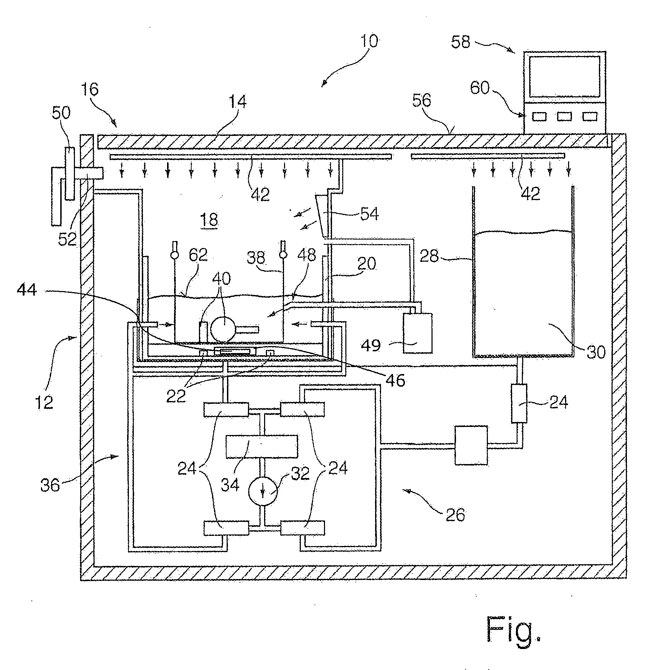

Die einzige Fig.1 zeigt einen stark schematisierten Schnitt durch ein erfindungsgemäßes maschinelles Reinigungs- und Desinfektionssystem **10**, beispielsweise für zahnärztliches Instrumentarium.

Das Reinigungs- und Desinfektionssystem 10 umfasst ein für ultraviolette (UV-) Strahlen sowie Infrarotstrahlen undurchlässiges Gehäuse **12**, mit einem Gehäusedeckel **14** und einer verschließbaren Zugangsöffnung **16** für das Einbringen und Entnehmen von zu reinigenden und zu desinfizierenden Gegenständen.

Das Gehäuse 12 umgrenzt einen Arbeitsraum **18** mit einer Ultraschallwanne **20**, die zwei Ultraschällquellen **22** aufweist. Die Ultraschallwanne 20 ist über ein mit steuerbaren Ventilen **24** versehenes Rohrleitungssystem **26** mit einem Vorratsbehältnis **28** für Wasser **30** verbunden. Das in dem Vorratsbehältnis 28 bevorratete Wasser 30 ist mittels einer Pumpe **32** durch das Rohrleitungssystem 26 in die Ultraschallwanne 20 beförderbar.
Eine dem Rohrleitungssystem 26 zugeordnete Heizeinrichtung **34** ermöglicht ein bedarfsweises Erhitzen des der Ultraschallwanne 20 zuzuführenden Wassers 30.

Das in der Ultraschallwanne 20 befindliche Wasser 30 ist in dem Rohrleitungssystem 26 entlang eines geschlossenen Kreislaufs **36** führbar, wodurch eine definierte Umwälzung des Wassers 30 in der Ultraschallwanne 20 ermöglicht wird. Ein definiertes Über- bzw. Durchströmen von in der Ultraschallwanne 20 in einem Einsatz **38** positionierten Gegenständen **40** mit dem Wasser 30 wird sichergestellt.

Oberhalb der Ultraschallwanne 20 sowie des Vorratsbehältnisses 28 ist jeweils ein UV-Strahler **42** zur Abgabe ultravioletter Strahlen angeordnet. Die Ultraschallwanne 20 ist aus reflektierendem Edelstahl gefertigt.

Das Reinigungs- und Desinfektionssystem 10 weist weiterhin ein zum Verdampfen von in der Arbeitskammer 18 enthaltenem Wassers ausgebildetes Heizelement **44** auf, das vorliegend in einem thermoresistenten Körper **46** eingelassen ist.

Der im Arbeitsraum 18 erzeugte Wasserdampf ist umwälzbar und kann den zu reinigenden Gegenständen 40 über eine Düse **48** als Dampfstrahl mit hoher Dampfstromrate zugeführt werden. Zum Umwälzen des Wasserdampfs dient ein Ventilator **49**.

Zur Begrenzung eines in der Arbeitskammer 18 herrschenden atmosphärischen Drucks weist das Gehäuse 12 einen mit einem einstellbaren Überdruckventil **50** versehenen Auslass **52** auf, über den bei Bedarf ein in dem Arbeitsraum 18 befindlicher Wasserdampf bei Erreichen eines voreingestellten Schwellendrucks aus dem Arbeitsraum 18 entweichen kann.

Der Arbeitsraum 18 weist weiterhin einen Infrarotstrahler **54** auf, mittels dessen der Arbeitsraum 18 sowie die darin befindlichen Gegenstände 40 kontrolliert erhitzt werden können.

Das Reinigungs- und Desinfektionssystem 10 weist eine an einer Außenseite **56** des Gehäuses 12 angeordnete Steuereinheit **58** mit einem Bedienfeld **60** auf.

Alle von der Steuereinheit erfassten (Betriebs-)Parameter werden in einem nicht näher wiedergegebenen Dokumentationszentrum elektronisch dokumentiert und können jederzeit zur Validierung der Reinigung und Desinfektion herangezogen werden.

Nachstehend wird das erfindungsgemäße Verfahren zur Reinigung und Desinfektion von Gegenständen näher erläutert.

In einem ersten Schritt werden die aufzubereitenden Gegenstände 40, z.B. medizinische Instrumente, über die mit dem Gehäusedeckel 14 verschließbare Zugangsöffnung 16 des Gehäuses 12 in die Ultraschallwanne 20 des Arbeitsraums 18 des Reinigungs- und Desinfektionssystems 10 eingebracht.

Nachfolgend wird die Zugangsöffnung 16 mittels des Gehäusedeckels 14 verschlossen und über das Bedienfeld 60 der Steuereinheit 58 ein in der Steuereinheit 58 definiertes Reinigungsprogramm ausgewählt und gestartet.

Dadurch werden die UV-Strahler 42 aktiviert. Diese erste Bestrahlungsphase wirkt direkt auf die Gegenstände und über die Reflektoren auch in Bereichen, die einer direkten Bestrahlung unzugänglich sind. Über die Pumpe 32 und das Rohrleitungssystem 26 wird nachfolgend Wasser 30 in die Ultraschallwanne 20 gepumpt, bis das Wasser 30 einen mittels des Reinigungsprogramms definierten Füllstand erreicht hat. Die von dem UV-Strahler 42 abgegebene UV-Strahlung wird dabei sowohl an der Wasseroberfläche 62 als auch an der Ultraschallwanne 20 reflektiert, so dass die zu reinigenden Gegenstände 40 umfassend gegenüber der UV-Strahlung exponiert werden.

Gleichzeitig werden die Ultraschallquellen 22 aktiviert und mit der Ultraschallreinigung der Gegenstände 40 wird begonnen.

Während der Ultraschallreinigung werden die Gegenstände 40 und alle in der Ultraschallwanne 20 angeordneten und vom Wasser 30 benetzten Komponenten durch den Ultraschall von Verunreinigungen befreit und somit laufend gereinigt. Hierbei werden größere Konglomerate von Verunreinigungen bzw. Keimen in kleinere Partikel aufgeteilt. Diese Partikel werden von dem Wasser 30, das mittels der Pumpe 24 im Rohrleitungssystem 26 zirkuliert, als Schwerstoffe mitgerissen, wobei Keime innerhalb des Arbeitsraums 18 von den Um-Strahlen erfasst und abgetötet werden.

Bei Erreichen einer in dem Reinigungsprogramm definierten Ultraschallreinigungszeit wird das in der Ultraschallwanne 20 befindliche Wasser 30 über das Rohrleitungssystem 26 mittels der Pumpe 32 bis auf ein in der Arbeitskammer verbleibendes (definiertes) Restvolumen des Wassers 30 entfernt, d.h. vorliegend für eine erneute Verwendung in das Vorratsbehältnis 28 befördert. Es schließt sich eine Bestrahlungsphase mit UV-Strahlung an, in der die gereinigten Gegenstände weiter UV-bestrahlt werden.

Gleichzeitig wird das elektrische Heizelement 44 aktiviert und das in dem Arbeitsraum 18 verbliebene Restvolumen des Wassers 30 wird aufgeheizt und zu gesättigtem Wasserdampf verdampft.

Der sich in dem Arbeitsraum 18 befindliche Wasserdampf wird bei einem Überschreiten eines vorbestimmten Drucks binnen Sekunden über den mit dem Überdruckventil 50 versehenen Auslass 52 aus dem Arbeitsraum 18 verdrängt. Die UV-Bestrahlung der Ultraschallwanne 20 bzw. der Gegenstände 40 wird während dieser Dampfdesinfektion der Gegenstände 40 fortgeführt.

Bei Erreichen einer durch das Reinigungsprogramm vorgegebenen Einwirkzeit des Sattdampfes wird die Dampfdesinfektion beendet und die in der Arbeitskammer 18 befindliche Dampfatmosphäre wird über ein nicht näher wiedergegebenes Belüftungssystem durch gefilterte Umgebungsluft ersetzt. Dadurch wird eine im Arbeitsraum 18 verbliebene Restfeuchtigkeit abgeführt und die gereinigten sowie desinfizierten Gegenstände 40 können, vorzugsweise nach ihrem Abkühlen, dem Reinigungs- und Desinfektionssystem trocken, d.h. ohne nennenswerte Restfeuchte, für einen ggf. unmittelbar nachfolgenden Einsatz, beispielsweise an einem Patienten, entnommen werden.

Die Erfindung betrifft ein Verfahren zur maschinellen Reinigung und Desinfektion von Gegenständen 40, insbesondere medizinischem und/oder zahnmedizinischem Instrumentarium und/oder Arbeitsmitteln. Erfindungsgemäß werden die Gegenstände 40 in einem Arbeitsraum 18 eines Reinigungs- und Desinfektionssystems 10 in Wasser 30 mit Ultraschall gereinigt, wobei das Wasser 30 nach dem Reinigen der Gegenstände 40 bis auf ein definiertes Restvolumen aus dem Arbeitsraum 18 entfernt wird. Das in dem Arbeitsraum 18 verbleibende Restvolumen des Wassers 30 wird nachfolgend mittels eines in dem Arbeitsraum 18 angeordneten Heizelements 44 in Wasserdampf umgewandelt. Die Gegenstände 40 werden in dem Arbeitsraum 18 mittels des Wasserdampfs thermisch desinfiziert, wobei eine in dem Arbeitsraum 18 befindliche Dampfatmosphäre nachfolgend zum Trocknen der Gegenstände 40 aus dem Arbeitsraum 18 entfernt wird.

## Patentansprüche

1. Verfahren zur maschinellen Reinigung und Desinfektion von Gegenständen (40), insbesondere medizinischem und/oder zahnmedizinischem Instrumentarium und/oder Arbeitsmitteln, bei dem die Gegenstände (40) in einem Arbeitsraum (18) eines Reinigungs- und Desinfektionssystems (10) in Wasser (30) mit Ultraschall gereinigt werden, wobei das Wasser (30) nach dem Reinigen der Gegenstände (40) bis auf ein definiertes Restvolumen aus dem Arbeitsraum (18) entfernt wird, wobei
das in dem Arbeitsraum (18) verbleibende Restvolumen des Wassers (30) nachfolgend mittels eines in dem Arbeitsraum (18) angeordneten Heizelements (44) in Wasserdampf umgewandelt wird, wobei die Gegenstände (40) in dem Arbeitsraum (18) mittels des Wasserdampfs thermisch desinfiziert werden und wobei
im Anschluss an die Dampfdesinfektion der Gegenstände eine in dem Arbeitsraum (18) befindliche Dampfatmosphäre aus dem Arbeitsraum (18) entfernt wird.

2. Verfahren nach Anspruch 1, **dadurch gekennzeichnet, dass** die Gegenstände (40) während der Ultraschallreinigung mit keimabtötenden UV-Strahlen bestrahlt werden.

3. Verfahren nach Anspruch 1 oder 2, **dadurch gekennzeichnet, dass** das Wasser (30) mittels eines elektrischen Heizelements, insbesondere mittels einer Heizwendel und/oder mittels eines Infrarotstrahlers (54) in Wasserdampf umgewandelt wird.

4. Verfahren nach einem der vorhergehenden Ansprüche, **dadurch gekennzeichnet, dass** die thermische Desinfektion der Gegenstände (40) über einen Zeitraum von zumindest 3 Minuten, vorzugsweise 5 Minuten, durchgeführt wird.

5. Verfahren nach einem der vorhergehenden Ansprüche, **dadurch gekennzeichnet, dass** der Wasserdampf eine Temperatur von 100° Celsius bis 140° Celsius, vorzugsweise von 100° bis 125° Celsius, ganz besonders bevorzugt von etwa 121 ° Celsius, aufweist.

6. Verfahren nach einem der vorhergehenden Ansprüche, **dadurch gekennzeichnet, dass** der Wasserdampf Sattdampf ist.

7. Verfahren nach einem der vorhergehenden Ansprüche, **dadurch gekennzeichnet, dass** der Wasserdampf mittels zumindest einer Düse (48) in Form eines auf die Gegenstände (40) gerichteten Dampfstrahls zugeführt wird.

8. Verfahren nach einem der vorhergehenden Ansprüche, **dadurch gekennzeichnet, dass** ein, sich im Arbeitsraum (18) durch die Dampfatmosphäre einstellender Druck durch ein, vorzugsweise einstellbares, Überdruckventil (50) oder durch eine, vorzugsweise in ihrem Strömungsquerschnitt einstellbare, Auslassöffnung am Arbeitsraum (18) begrenzt wird.

9. Verfahren nach einem der vorhergehenden Ansprüche, **dadurch gekennzeichnet, dass** der Arbeitsraum (18) und die Gegenstände (40), vorzugsweise durch Bestrahlen mit Infrarotstrahlen, vor der Dämpfdesinfektion auf eine Temperatur von über 100° Celsius, insbesondere auf eine Temperatur von ca. 121° Celsius erhitzt werden.

10. Verfahren nach einem der vorhergehenden Ansprüche, **dadurch gekennzeichnet, dass** die Gegenstände (40) vor der Ultraschallreinigung, insbesondere vor einem Benetzen der Gegenstände (40) mit dem Wasser, und/oder zumindest bis zum Ende der thermischen Desinfektion mit den UV-Strahlen bestrahlt werden.

11. Verfahren nach einem der vorhergehenden Ansprüche, **dadurch gekennzeichnet, dass** Gegenstände (40) mit einem von außen zugänglichen Hohlraum, insbesondere (zahn-) medizinischen Hand- und Winkelstücke, an eine Spüleinrichtung angeschlossen werden, wobei die Gegenstände (40) mittels der Spüleinrichtung während der Ultraschallreinigung der Gegenstände (40) mit dem Wasser (30) und während der thermischen Desinfektion mit dem Wasserdampf durchspült werden.

12. Verfahren nach einem der vorhergehenden Ansprüche, **dadurch gekennzeichnet, dass** Reinigungs- und Desinfektionsparameter, insbesondere eine jeweilige Einwirkzeit des Ultraschalls und/oder des Dampfes von einer Steuereinheit (58) gesteuert werden, wobei die einzelnen Parameter über ein der Steuereinheit (58) zugeordnetes Bedienelement (60) frei vorgegeben werden und/oder wobei die Parameter durch Aufrufen eines in der Steuereinheit (58) definierten, vorzugsweise spezifisch auf die Reinigung der Gegenstände (40) ausgelegten, Reinigungsprogramms vorgegeben werden und zumindest eine Auswahl der Parameter dokumentiert werden.

## Claims

1. A method for machine cleaning and disinfecting objects (40), in particular medical and/or dental instruments and/or work equipment, in which the objects (40) are ultrasonically cleaned in water (30) in a working area (18) of a cleaning and disinfection system (10), wherein after cleaning of the objects (40) the water (30) is removed from the working area (18) up to a defined residual volume, wherein
the residual volume of water (30) remaining in the working area (18) is subsequently converted into water vapour by means of a heating element (44) arranged in the working area (18), wherein the objects (40) in the working area (18) are thermally disinfected by means of the water vapour and wherein subsequent to the vapour disinfection of the objects a vapour atmosphere in the working area (18) is removed from the working area (18).

2. A method according to claim 1, **characterised in that** the objects (40) are irradiated with germicidal UV rays during the ultrasonic cleaning.

3. A method according to claim 1 or 2, **characterised in that** the water (30) is converted into water vapour by means of an electric heating element, in particular by means of a heating coil and/or by means of an infrared emitter (54).

4. A method according to any one of the preceding claims, **characterised in that** the thermal disinfection of the objects (40) is carried out over a period of at least 3 minutes preferably 5 minutes.

5. A method according to any one of the preceding claims, **characterised in that** the water vapour has a temperature of 100° Celsius to 140° Celsius, preferably 100° to 125° Celsius, especially preferably about 121 ° Celsius.

6. A method according to any one of the preceding claim, **characterised in that** the water vapour is saturated steam.

7. A method according to any one of the preceding claims, **characterised in that** the water vapour is supplied by means of at least one nozzle (48) in the form of a vapour jet directed onto the objects (40).

8. A method according to any one of the preceding claims, **characterised in that** a pressure developing in the working area (18) as a result of the vapour atmosphere is limited by a preferably adjustable pressure relief valve (50) or by an outlet opening, preferably having an adjustable flow cross-section, at the working area (18).

9. A method according to any one of the preceding claims, **characterised in that** the working area (18) and the objects (40) are heated, preferably by irradiation with infrared rays, before the vapour disinfection to a temperature of above 100°Celcius, in particular to a temperature of approximately 121° Celsius.

10. A method according to any one of the preceding claims, **characterised in that** the objects (40) are irradiated with the UV rays before the ultrasonic cleaning, in particular before the objects (40) are wetted with the water, and/or at least to the end of the thermal disinfection.

11. A method according to any one of the preceding claims, **characterised in that** objects (40) having an externally accessible cavity, in particular dental and medical hand pieces and angle pieces are connected to a flushing device, wherein by means of the flushing device the objects (40) are flushed through with the water (30) during the ultrasonic cleaning of the objects (40) and with the water vapour during the thermal disinfection.

12. A method according to any one of the preceding claims, **characterised in that** cleaning and disinfection parameters, in particular a particular residence time of the ultrasound and/or of the vapour are controlled by a control unit (58), wherein the individual parameters are freely preset via an operator control element (60) associated with the control unit (58) and/or wherein the parameters are preset by calling up cleaning programme defined in the control unit (58), preferably specifically designed for the cleaning of objects (40), and at least a selection of the parameters is documented.

## Revendications

1. Procédé de nettoyage et de désinfection automatiques d'objets (40), notamment d'appareillages et/ou d'instruments médicaux et/ou dentaires, dans lequel lesdits objets (40) sont nettoyés aux ultrasons, dans de l'eau (30), dans une chambre de travail (18) d'un système (10) de nettoyage et de désinfection, l'eau (30) étant évacuée de ladite chambre de travail (18) jusqu'à un volume résiduel bien défini, à l'issue du nettoyage desdits objets (40), sachant
que le volume résiduel de l'eau (30), subsistant dans la chambre de travail (18), est ensuite transformé en vapeur d'eau au moyen d'un élément chauffant (44) logé dans ladite chambre de travail (18), lesdits objets (40) étant alors désinfectés thermiquement à l'aide de la vapeur d'eau, dans ladite chambre de travail (18), et sachant
qu'une atmosphère de vapeur, située dans la chambre de travail (18), est éliminée de ladite chambre de travail (18) dans l'enchaînement direct de la désinfection desdits objets à la vapeur.

2. Procédé selon la revendication 1, **caractérisé par le fait que** les objets (40) sont exposés à des rayonnements UV bactéricides au cours du nettoyage aux ultrasons.

3. Procédé selon la revendication 1 ou 2, **caractérisé par le fait que** l'eau (30) est transformée en vapeur d'eau au moyen d'un élément chauffant électrique, en particulier au moyen d'un serpentin de chauffage et/ou au moyen d'un diffuseur d'infrarouges (54).

4. Procédé selon l'une des revendications précédentes, **caractérisé par le fait que** la désinfection thermique des objets (40) est exécutée durant une période d'au moins 3 minutes, de préférence de 5 minutes.

5. Procédé selon l'une des revendications précédentes, **caractérisé par le fait que** la vapeur d'eau présente une température de 100 °Celsius à 140 °Celsius, de préférence de 100 °Celsius à 125 °Celsius, et d'environ 121 °Celsius avec préférence particulière.

6. Procédé selon l'une des revendications précédentes, **caractérisé par le fait que** la vapeur d'eau est de la vapeur saturée.

7. Procédé selon l'une des revendications précédentes, **caractérisé par le fait que** la vapeur d'eau est délivrée, à l'aide d'au moins une buse (48), sous la forme d'un jet de vapeur dirigé vers les objets (40).

8. Procédé selon l'une des revendications précédentes, **caractérisé par le fait qu'**une pression, s'instaurant dans la chambre de travail (18) sous l'effet de l'atmosphère de vapeur, est limitée par l'intermédiaire d'une vanne de surpression (50) de préférence réglable, ou par l'intermédiaire d'un orifice de sortie pratiqué sur ladite chambre de travail (18), et dont la section transversale d'écoulement est de préférence réglable.

9. Procédé selon l'une des revendications précédentes, **caractérisé par le fait que** la chambre de travail (18) et les objets (40) sont chauffés préalablement à la désinfection à la vapeur, de préférence par exposition à des rayonnements infrarouges, jusqu'à une température excédant 100 °Celsius et, en particulier, jusqu'à une température d'environ 121 °Celsius.

10. Procédé selon l'une des revendications précédentes, **caractérisé par le fait que** les objets (40) sont exposés aux rayonnements UV préalablement au nettoyage par ultrasons, en particulier préalablement à une imprégnation desdits objets (40) par l'eau, et/ou au moins jusqu'à la fin de la désinfection thermique.

11. Procédé selon l'une des revendications précédentes, **caractérisé par le fait que** des objets (40) munis d'une cavité accessible de l'extérieur, en particulier des pièces à main et des pièces coudées médicales (médico-dentaires), sont raccordés à un dispositif de rinçage, lesdits objets (40) étant alors rincés de part et part au moyen dudit dispositif de rinçage, par l'eau (30) au cours du nettoyage aux ultrasons desdits objets (40), et par la vapeur d'eau au cours de la désinfection thermique.

12. Procédé selon l'une des revendications précédentes, **caractérisé par le fait que** des paramètres de nettoyage et de désinfection, en particulier une durée d'action respective des ultrasons et/ou de la vapeur, sont pilotés par une unité de commande (58), les paramètres individuels étant librement préétablis par l'intermédiaire d'un élément d'actionnement (60) affecté à ladite unité de commande (58), et/ou lesdits paramètres étant préétablis par interrogation d'un programme de nettoyage bien défini dans ladite unité de commande (58) et, de préférence, spécifiquement conçu pour le nettoyage des objets (40), au moins une sélection desdits paramètres étant documentée.
